# EUROPEAN PATENT APPLICATION

(11) **EP 0 821 959 A2**
(43) Date of publication of application: **04.02.1998**
(21) Application number: 97305594.0
(22) Date of filing: 25.07.1997
(51) Int. Cl.: A61K 31/44

(54) **Use of 3-(4-hexyloxy-1,2,5-thiadiazol-3-yl)-1,2,5,6-tetrahydro-1-methylpyridine for treating nicotine withdrawal**

(30) Priority: 01.08.1996 US 22902 P
(71) Applicant: ELI LILLY AND COMPANY, Indianapolis, Indiana 46285 (US)
(72) Inventor: Rasmussen, Kurt, Fishers, Indiana 46038 (US)
(74) Representative: Hudson, Christopher Mark

(57) **Abstract**

The present invention provides a method for treating a nicotine withdrawal in a mammal using a compound of the Formula I

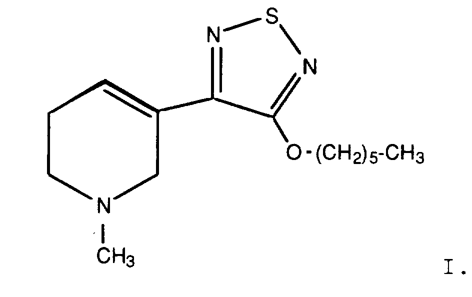

## Description

This invention provides a method for using 3-(4-hexyloxy-1,2,5-thiadiazol-3-yl)-1,2,5,6-tetrahydro-1-methylpyridine for the treatment nicotine withdrawal and alleviation of the craving for a tobacco product.

Over 25 years ago, the Surgeon General issued a report linking cigarette smoking to cancer, heart disease, respiratory disease and other conditions. Despite such information being available to the public, cigarette smoking remains a significant preventable cause of death in the United States and other developed countries.

Benowitz, *N. Eng. J. Med.* 319:20, 1318-1330 (November 17, 1988) notes that many people who smoke cigarettes would like to quit but cannot because they are addicted to the psychoactive drug that is the dependence-producing constituent of tobacco, nicotine.

Benowitz notes that nicotine may also contribute to the diseases for which smoking is a risk factor, particularly heart disease. Nicotine is also present in other tobacco products that are smoked or chewed, which are also addictive and associated with heart, lung, and other serious disease states.

Pharmacologic therapies are known to help those addicted to nicotine. Receptor antagonists such as mecamylamine have been used to reduce the satisfaction obtained from tobacco use. Unfortunately, this therapy has the short term effect of increasing tobacco consumption to overcome the receptor antagonism as well as other undesirable side effects.

Non-receptor antagonists have also been used, such as clonidine to reduce the craving for tobacco and other tobacco related withdrawal symptoms. According to Benowitz, one recent study using clonidine treatment for six weeks was found to be more effective than placebo, but only for women.

Benowitz reports that the most effective treatment thus far has been nicotine substitution therapy, using nicotine gum, or other nicotine forms to slowly wean individuals from the addiction to nicotine and the tobacco products containing nicotine. Unfortunately, the nicotine substitution therapy involves the administration of the psychoactive constituent of tobacco which has been identified as a contributor to the diseases associated with smoking. Nicotine substitution must be tapered, frequently leading to nicotine withdrawal and subsequent relapse to smoking. There is a need for a therapy having a desirable side effect profile, to relieve nicotine withdrawal symptoms, including the long term cravings for nicotine.

Sauerberg *et al.* in U.S. Patent 5,043,345 ('345) disclose the 3-(4-hexyloxy-1,2,5-thiadiazol-3-yl)-1,2,5,6-tetrahydro-l-methylpyridine compound which Applicants have discovered is useful for treating nicotine withdrawal. The compounds in the '345 patent are taught to be useful in treating Alzheimer's disease, and stimulating the cognitive function of the forebrain and hippocampus of mammals. There is no disclosure in the patent of using the compounds to treat nicotine withdrawal.

We have discovered that 3-(4-hexyloxy-1,2,5-thiadiazol-3-yl)-1,2,5,6-tetrahydro-1-methylpyridine, thought to be a muscarinic agonist, can be useful for treating can be useful for treating a condition which is a response produced by cessation and withdrawal from the use of nicotine. The present invention relates to a method of treating nicotine withdrawal. More specifically, the invention provides a method of treating nicotine withdrawal in humans using 3-(4-hexyloxy-1,2,5-thiadiazol-3-yl)-1,2,5,6-tetrahydro-1-methylpyridine.

As noted hereinbefore, the compounds employed in the method of the present invention are known. Methods of preparing the compounds, as well as pharmaceutical formulations containing the compounds, are taught by Sauerberg in U.S. Pat. No. 5,043,345 herein incorporated by reference in its entirety.

The presently claimed invention provides a method for treating a condition which is a response produced by cessation and withdrawal from the use of nicotine, comprising administering an effective amount of a compound of Formula I: 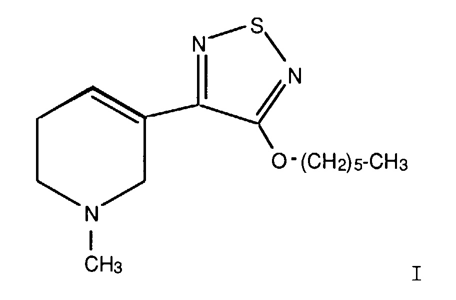 or a pharmaceutically acceptable salt or solvate thereof.

As used herein, the term "nicotine withdrawal" or "cessation and withdrawal from the use of nicotine" shall refer to a condition resulting from discontinued consumption of tobacco products and consequently, a result of discontinued consumption of nicotine. Such nicotine withdrawal conditions are characterized in the DSM-IV-R. Diagnostic and Statistical Manual of Mental Disorders, Revised, 3rd Ed. (1994). The DSM-IV-R was prepared by the Task Force on Nomenclature and Statistics of the American Psychiatric Association, and provides clear descriptions of diagnostic catagories. The skilled artisan will recognize that there are alternative nomenclatures, nosologies, and classification systems for pathologic psychological conditions and that these systems evolve with medical scientific progress.

Therefore, the term "cessation and withdrawal from the use of nicotine" shall include, but is not limited to, the following conditions characterized in the DSM-IV-R: Nicotine Withdrawal; Nicotine-Related Disorder Not otherwise Specified; Nicotine Dependence, with physiological dependence; Nicotine Dependence, without physiological dependence; Nicotine Dependence, Early Full Remission; Nicotine Dependence, Early Partial Remission; Nicotine Dependence, Sustained Full Remission; and Nicotine Dependence, Sustained Partial Remission; Nicotine Dependence, On Agonist Therapy.

The discontinued use of tobacco products, all of which contain nicotine, results in the onset of nicotine withdrawal syndrome. Individuals typically suffer the symptoms of nicotine withdrawal resulting from the discontinued use of tobacco in any form, including, but not limited to smoking of cigarette, cigar, or pipe tobacco, or the oral or intranasal ingestion of tabacco or chewing tobacco. Such oral or intranasal tobacco includes, but is not limited to snuff and chewing tobacco. The cessation of nicotine use or reduction in the amount of nicotine use, is often followed within 24 hours by dysphoric, depressed mood; insomnia; irritability, frustration or anger; anxiety; difficulty concentrating; restlessness; decreased heart rate; increased appetite or weight gain. These symptoms often cause clinically significant distress or impairment in social, occupational, or other important areas of functioning. The present invention is most preferably used to alleviate symptoms attributed to nicotine withdrawal when such symptoms are not due to a general medical condition and are not better accounted for by another medical disorder.

The method of the present invention is preferably administered in connection with and/or subsequent to an educational and/or behavioral modification program to ensure continued abstinence from tobacco products. The method of the present invention is also highly beneficial to such programs by alleviating the suffering experienced from the nicotine withdrawal over the course of such programs. Therefore, the programs can be more effective by focusing on educational and behavioral modification goals, further reducing the incidence of program non-completion.

The method of this invention further provides a method for alleviating the desire of the subject to relapse. The administration of an effective amount of xanomeline to a subject in need or desirous thereof is thought to block the physiological receptor responsible for triggering a relapse. Therefor, the subject is less likely to resume smoking after nicotine consumption has ceased.

The term "effective amount", as used herein, represents an amount of compound necessary to prevent or treat a human susceptible to suffering from a nicotine withdrawal following administration to such human. The active compound is effective over a wide dosage range. For example, dosages per day will normally fall within the range of about 0.005 to about 500 mg/kg of body weight. In the treatment of adult humans, the range of about 0.05 to about 100 mg/kg, in single or divided doses, is preferred. However, it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration, and therefore the above dosage ranges are not intended to limit the scope of the invention in any way. While the present compound may be administered orally to humans susceptible to or suffering from nicotine withdrawal, the compound is particularly well suited to be administered transdermally. When the compound is deleivered transdermally, it is preferred that the effective amount is from about 10mg to about 100mg per day delivery of base compound. It is especially preferred that such patch delivers an effective amount for about one to seven days.

The compound may further be delivered by a variety of other pharmaceutically accepted routes including, but in no way limited to parenterally, subcutaneous, intranasal, intramuscular and intravenous routes. Such formulations may be designed to provide delayed or controlled release using formulation techniques which are known in the art.

As used herein the term "treating" includes prophylaxis of a physical and/or mental condition or amelioration or elimination of the developed physical and/or mental condition once it has been established or alleviation of the characteristic symptoms of such condition.

In addition, 3-(4-hexyloxy-1,2,5-thiadiazol-3-yl)-1,2,5,6-tetrahydro-1-methylpyridine has been found to have a favorable profile of activity in a number of in vitro binding assays, designed to measure the degree of binding to neural receptors.

The compounds employed in the invention are not believed to act via the GABA/benzodiazepine, serotonin, or dopamine receptor systems in humans. Rather, the activity of the present compound as a treatment for nicotine withdrawal is believed to be based upon modulation of muscarinic cholinergic receptors. However, the mechanism by which the present compound functions is not necessarily the mechanism stated *supra.,* and the present invention is not limited by any mode of operation.

The usefulness of the compound for treating a condition resulting from cessation and withdrawal from the use of nicotine can be supported by the following study.

### I. Auditory Startle Response.

Male Long Evans rats (Harlan Sprague Dawley) are individually housed in a controlled environment on a 12 hour light-dark cycle. The rats are given free access to food and water. All treatment groups contain from 8 to 10 rats.

The rats are anesthetized with halothane and Alzet osmotic minipumps (Alza Corporation, Palo Alto, California) are implanted subcutaneously. Nicotine ditartrate is dissolved in physiological saline. Pumps are filled with nicotine ditartrate (6 mg/kg base/day) or the appropriate vehicle. Twelve days following implantation of pumps, rats are anesthetized with halothane and the pumps are removed.

The auditory Startle Response is observed.

The sensory motor reactions [auditory startle response (peak amplitude, Vₘₐₓ)] of individual rats are recorded using San Diego Instruments startle chambers (San Diego, Calif.). Startle sessions consist of a 5 minute adaptation period at background noise level of 70 +/- 2dBA immediately followed by 25 presentations of auditory stimuli (120 +/-3 dBA noise, 50 ms duration) presented at 8 second intervals. Peak startle amplitudes are averaged for all 25 presentations of stimuli for each session. Auditory startle responding is evaluated daily at 24 hour intervals on days 1-4 following nicotine withdrawal.

The 3-(4-hexyloxy-1,2,5-thiadiazol-3-yl)-1,2,5,6-tetrahydro-l-methylpyridine compound is administed at six doses about 60 minutes before startle testing each day.

It will be appreciated that the compound of formula (I) may be isolated per se or may be converted to an acid addition salt using conventional methods.

The compound has an IC₅₀ of less than 1 mM in the ³H-QNB binding assay described by Yamamura, HI and Snyder, SH in Proc.Nat.Acad.Sci. USA 71 1725 (1974) indicating that it has muscarinic-cholinergic activity.

The compounds employed in the invention are not believed to act via the GABA/benzodiazepine, 5HT1A, or D1 receptor systems in humans. Rather, the activity of the present compound as a treatment for nicotine withdrawal is believed to be based upon modulation of muscarinic cholinergic receptors. However, the mechanism by which the present compounds function is not necessarily the mechanism stated *supra.,* and the present invention is not limited by any mode of operation.

Xanomeline has been studied using accepted pharmacological methods such as oxotremorine-M verses N-methylscopolamine binding studies (Freedman et al. Br. J. Pharmacology, **93**:437-445 (1988). Xanomeline inhibited the binding of ³H-oxotremorine-M with an inhibition costant (Kᵢ) of 2nM. The binding of the muscarinic ml antagonist ligand, ³H-pirenzepine, to ml receptors in hippocampus and ³H-quinuclidinyl benzilate to m2 receptors in brain stem was inhibited with Kᵢ values of 5 and 24 nM, respectively.

Muscarinic agonists stimulate the formation of cAMP up to 10 fold in CHO m4 cells treated with pertussisi toxin and the pharmacology is consistent with the mediation by m4 receptors. Eckols K. Soc. Neurosci Abstr., **21**:2040 (1995). In this assay, xanomeline efficaciously and potently stimulated the formation of cAMP. Such studies suggest that xanomeline predominantly activates m1 and m4 receptors.

The 3-(4-hexyloxy-1,2,5-thiadiazol-3-yl)-1,2,5,6-tetrahydro-1-methylpyridine compound will normally be administered orally or by injection and, for this purpose, it is usually employed in the form of a pharmaceutical composition.

Accordingly, pharmaceutical compositions comprising 3-(4-hexyloxy-1,2,5-thiadiazol-3-yl)-1,2,5,6-tetrahydro-1-methylpyridine, as active ingredient associated with a pharmaceutically acceptable carrier may be prepared. In making the compositions of the invention conventional techniques for the preparation of pharmaceutical compositions may be used. For example, the active ingredient will usually be mixed with a carrier, or diluted by a carrier, or enclosed within a carrier which may be in the form of a capsule, sachet, paper or other container. When the carrier serves as a diluent, it may be solid, semi-solid or liquid material which acts as a vehicle, excipient or medium for the active ingredient. The active ingredient can be adsorbed on a granular solid container for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient.

The following Examples are studies to establish the usefulness of the named compounds for treating such nicotine withdrawal.

### Example 1

### Human Clinical Trials

The activity of 3-(4-hexyloxy-1,2,5-thiadiazol-3-yl)-1,2,5,6-tetrahydro-1-methylpyridine for treating or alleviating nicotine withdrawal can be demonstrated by human clinical trials. The study was designed as a double-blind, parallel, placebo-controlled multicenter trial. The subjects were randomized into four groups, placebo and 25, 50, and 75 mg tid of test compound. The dosages were administered orally with food. Subjects were observed at four visits to provide baseline measurements. Visits 5-33 served as the treatment phase for the study.

During the visits, subjects are observed for signs of agitation, mood swings, and concentration abilities. Each of these behaviors are indicative of the effect of the test compound on nicotine withdrawal.

Treatment groups are compared with respect to the number and percent of subjects who ever had the symptom during the double-blind portion of the study (visits 5 through 33), at a severity that was worse than during the baseline visits (1 through 4).

## Claims

1. A method for treating a condition resulting from the cessation and withdrawal of nicotine comprising administering to a mammal in need of or desiring such treatment, an effective amount of a compound of Formula I: 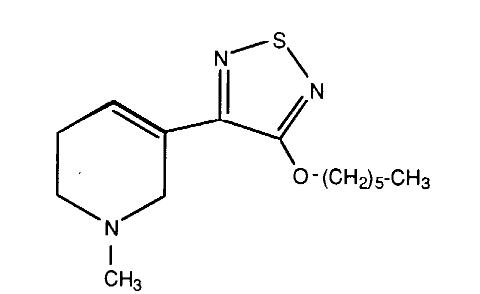 or a pharmaceutically acceptable salt thereof.

2. A method of **Claim 1** wherein the nicotine use is the smoking of tobacco.

3. A method of **Claim 2** wherein the nicotine use is the smoking of cigarettes.

4. A method of **Claim 1** wherein the condition is selected from the group consisting of Nicotine Withdrawal; Nicotine-Related Disorder Not otherwise Specified; Nicotine Dependence, with physiological dependence; Nicotine Dependence, without physiological dependence; Nicotine Dependence, Early Full Remission; Nicotine Dependence, Early Partial Remission; Nicotine Dependence, Sustained Full Remission; Nicotine Dependence, Sustained Partial Remission; and Nicotine Dependence, On Agonist Therapy.

5. A method of **Claim 4** wherein the condition is Nicotine Withdrawal.

6. A method of **Claim 1** where in the nicotine use is the oral ingestion of tobacco.

7. A method of **Claim 1** wherein the effective amount is from 1 mg/kg to about 100 mg/kg per day.

8. A method of **Claim 7** wherein the effective amount is from about 10 mg/kg to about 100 mg/kg per day.

9. A method of **Claim 1** wherein the effective amount is delivered using a transdermal patch.

10. A method of **Claim 9** wherein the transdermal patch delivers from about 10 to about 100 mg of base compound per day.

11. A method of **Claim 10** wherein the transdermal patch delivers an effective amount for one (1) to seven (7) days.

12. A method of **Claim 1** wherein the condition is relapse or the urge to relapse.

13. Use of a compound of Formula I: 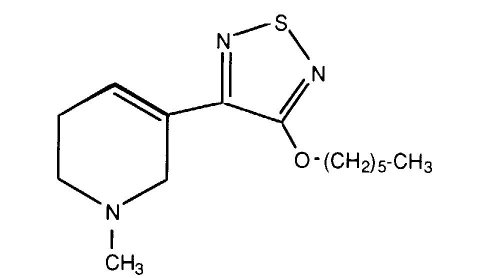 or a pharmaceutically acceptable salt thereof for the manufacture of a medicament for treating a condition resulting from the cessation and withdrawal of nicotine.

14. A Use of **Claim 13** wherein the nicotine use is the smoking of tobacco.

15. A use of **Claim 13** wherein the condition is selected from the group consisting of Nicotine Withdrawal; Nicotine-Related Disorder Not otherwise Specified; Nicotine Dependence, with physiological dependence; Nicotine Dependence, without physiological dependence; Nicotine Dependence, Early Full Remission; Nicotine Dependence, Early Partial Remission; Nicotine Dependence, Sustained Full Remission; Nicotine Dependence, Sustained Partial Remission; and Nicotine Dependence, On Agonist Therapy.

16. A method of **Claim 15** wherein the condition is Nicotine Withdrawal.

17. A use of **Claim 13** wherein the compound of Formula I is delivered using a transdermal patch.

18. A use of **Claim 17** wherein the transdermal patch delivers from about 10 to about 100 mg of base compound per day.

19. A use of **Claim 18** wherein the transdermal patch delivers an effective amount for one (1) to seven (7) days.
